# EUROPÄISCHE PATENTSCHRIFT

(11) **EP 1 929 964 B1**
(45) Veröffentlichungstag und Bekanntmachung des Hinweises auf die Patenterteilung: **21.10.2009**
(21) Anmeldenummer: 06025111.3
(22) Anmeldetag: 05.12.2006
(51) Int. Cl.: A61B 17/17, A61M 1/00, A61B 17/00

(54) **Halterung für einen Knochenbohrer und Knochenbohrer-Halterungssystem**
Bone drill holder and bone drill holding system
Support pour instrument de perçage et système de fixation de cet instrument

(43) Veröffentlichungstag der Anmeldung: 11.06.2008
(73) Patentinhaber: BrainLAB AG, 85622 Feldkirchen (DE)
(72) Erfinder: Plassky, Norman, 99099 Erfurt (DE); Neubauer, Timo, 85586 Poing (DE); Fessler, Julia, 85622 Feldkirchen (DE)
(74) Vertreter: Schwabe - Sandmair - Marx

(56) Entgegenhaltungen:
- EP-A- 0 923 906
- WO-A-03/096870
- US-A- 5 312 408
- US-A1- 2004 082 960

## Beschreibung

Die Erfindung betrifft eine Halterung für einen Knochenbohrer und ein Knochenbohrer-Halterungssystem.

Insbesondere betrifft sie eine Halterung für einen Knochenbohrer, die an einem Knochen anzubringen ist, speziell an dem Kopf eines Oberschenkelknochens (Gelenkkopf).

Um die Femurkomponente während der Oberflächen-Wiederherstellung in der Hüftchirurgie anzubringen, muss der Femurknochen präpariert werden, so dass er eine spezielle Form erhält. Einer der wichtigsten Aspekte während der Präparierung des Femurknochens liegt darin, jedwede Art von Einkerbungen oder Schwächungen des Gelenkhalses zu vermeiden, da durch solche Schwächungen oder Einkerbungen Risse und Brüche hervorgehen können. Die Femur-Implantatkomponente hat eine symmetrische Innenform mit einem Mittelstift, und es ist wichtig, die Position und Ausrichtung des Lochs für diesen Mittelstift genau zu definieren und zu realisieren. Während der Oberflächen-Wiederherstellungsbehandlung wird eine Pilotbohrung für den Mittelstift des Implantats hergestellt, und es ist ebenso wichtig, die Pilotbohrung so genau wie möglich zu setzen, da alle weiteren Bearbeitungsschritte darauf aufbauen.

Zur Unterstützung dieses Bohrvorgangs sind verschiedene Werkzeuge eingesetzt worden, um den Eintrittspunkt und die Achse für die Bohrung zu definieren. Aus der US 6,156,069 geht eine Greifvorrichtung hervor, die am Oberschenkelhals angreift und den Bohrer führen kann. Die US 6,595,999 B2 offenbart eine als Rahmen angebrachte Bohrerhalterung bei der ein Messfühler zum Sicherstellen der Positionierung vorhanden ist. Aus der US 6,595,999 B1 ist ferner auch noch eine direkte Befestigung am Femurkopf bzw. eine Befestigung an einer planen Fläche des Femurkopfes, abfolgend auf eine Fräsprozedur, bekannt.

Die EP 0 923 906 A2 (Basis für den Oberbegriff des Anspruchs 1) betrifft eine Vorrichtung, um die Ausrichtung für das Einführen eines Instruments, wie eines Bolzens, einer Stange, eines Nagels, einer Schraube, eines Drahtes, eines Bohrers oder eines anderen Implantats in Knochengewebe unter Verwendung von Röntgenstrahlung oder Durchleuchtung oder dergleichen festzustellen, um Frakturen oder andere Knochengewebeschäden zu stabilisieren.

Es ist die Aufgabe der vorliegenden Erfindung, eine Knochenbohrerhalterung, insbesondere für die oben angegebenen Einsatzzwecke, zu schaffen, welche eine optimale Anordnung und Ausrichtung der vorzunehmenden Knochenbohrung und damit auch des Oberflächen-Wiederherstellungsimplantates ermöglicht. Insbesondere soll dabei eine stabile Verbindung mit dem Knochen erzielt werden, ohne dass dieser verletzt oder eingekerbt wird.

Diese Aufgabe wird erfindungsgemäß durch eine Knochenbohrer-Halterung gemäß dem Patentanspruch 1 gelöst. Die Unteransprüche definieren bevorzugte Ausführungsformen der Erfindung.

Erfindungsgemäß hat die Knochenbohrerhalterung einen Befestigungsteil zur Befestigung am Knochen, wobei dieser Befestigungsteil mit einer Unterdruck-Ansaugung am Knochen befestigt wird. Mit anderen Worten saugt sich der Befestigungsteil am Knochen fest, ohne in diesen eingreifen zu müssen. Dies hat vielfältige Vorteile.

Zunächst gestattet die vorliegende Erfindung eine feste Ausrichtung der Bohrerhalterung, und der Chirurg kann die Bohrung in der definierten Richtung durchführen. Das Risiko des Abrutschens des Bohrers von der Knochenoberfläche wird stark verringert.

Ferner werden keine zusätzlichen Elemente (Stifte, Schrauben, Greifer etc.) zum Anbringen der Halterung an dem Knochen notwendig. Die stabile Verbindung mit dem Knochen wird durch eine Druckdifferenz hergestellt, ebenso wird dadurch der Bohrwinkel fixiert. Einerseits macht es der Verzicht auf Schrauben oder Stifte möglich, auf entsprechende Knocheneinkerbungen bzw. Knochenbeschädigungen zu verzichten, die Startpunkte für Brüche oder Risse bilden können, andererseits werden durch Vermeidung von Einmalprodukten zur Befestigung die Kosten gesenkt. Es wird auch Zeit eingespart, weil die Herstellung des Unterdrucks nur ein Absaugen von Luft umfasst, was bei geeigneter Ausführung sehr schnell geht. Außerdem wird keine zusätzliche Vorbereitung des Knochens (zum Beispiel ein Abfräsen) notwendig. Die Handhabung ist einfach und nicht invasiv, und sehr gut mit einer Navigationsunterstützung kombinierbar, so dass zusätzliche Messfühler zur Verifizierung der Ausrichtung ebenfalls entfallen können.

Gemäß einer Ausführungsform der Erfindung weist der Befestigungsteil der Halterung einen Befestigungs-Unterdruckraum oder Befestigungs-Unterdruckbereich auf, sowie eine Ventileinrichtung, über die der Druck im Befestigungs-Unterdruckraum oder Befestigungs-Unterdruckbereich reguliert werden kann. Die Abdichtung des Befestigungs-Unterdruckraumes kann durch eine am Knochen angreifende Dichteinrichtung erfolgen, insbesondere durch eine Dichtschürzenanordnung um den Befestigungs-Unterdruckraum oder Befestigungs-Unterdruckbereich.

Es ist möglich, am Befestigungsteil einen Bohrerführungsteil anzuordnen, der gegenüber dem Befestigungsteil beweglich ist. Der Bohrerführungsteil kann einen auf dem Befestigungsteil verschieblichen und abgedichteten Aufsatz mit einer Bohrerführung bilden. Ferner kann er einen Führungs-Unterdruckraum oder Führungs-Unterdruckbereich aufweisen, der bei der Ausbildung eines Unterdrucks die Lage des Bohrerführungsteils gegenüber dem Befestigungsteil fixiert.

Der Unterdruck kann in verschiedener Art und Weise eingesetzt werden. Einerseits besteht die Möglichkeit, die Befestigungs- und Führungs-Unterdruckräume oder -Unterdruckbereiche miteinander zu verbinden, so dass sie einen einzigen Unterdruckbereich bzw. Unterdruckraum bilden. In diesem Fall würde die Herstellung des Unterdrucks sowohl für die Befestigung der Halterung am Knochen als auch für die Ausrichtung und Fixierung der Bohrerführung dienen.

In einem anderen Fall wären die Unterdruckräume oder Unterdruckbereiche für die Befestigung und die Führung voneinander separiert, so dass die Befestigung und die Fixierung der Bohrerausrichtung separat erfolgen kann, durch das Anlegen separater Unterdrücke. Es ist grundsätzlich auch denkbar, dass nur die Ausrichtung bzw. Fixierung des Bohrerführungsteils per Unterdruck erfolgt.

Für den Bohrerführungsteil kann eine separate Ventileinrichtung bereitgestellt werden, über die der Druck im Führungs-Unterdruckraum oder Führungs-Unterdruckbereich reguliert werden kann.

Die Erfindung umfasst ferner mehrere Kombinationen der erfindungsgemäßen Knochenbohrerhalterung mit weiteren medizintechnischen Geräten oder Vorrichtungen. Insbesondere umfasst sie ein Knochenbohrer-Halterungssystem mit einer Knochenbohrerhalterung, wie sie vorher in unterschiedlichen Ausführungsformen beschrieben wurde, und mit einem Handgriff, mit dem die Halterung mittels einer

Bohrerführung gehalten werden kann. Der Handgriff kann mit einer Unterdruckquelle und mit der Unterdruck-Ansaugung des Befestigungsteils verbunden sein, und in spezieller Ausführung können dem Handgriff Steuerungsvorrichtungen zum Steuern des Unterdrucks zugeordnet sein. Es ist auch möglich, mit dem Handgriff nicht nur die Ansaugung des Befestigungsteils sondern auch die Fixierung des Bohrerführungsteils durch Unterdruck zu steuern, oder eine bzw. beide dieser Steuerungen unabhängig voneinander durchzuführen.

Ein weiteres Knochenbohrer-Halterungssystem mit einer Knochenbohrerhalterung gemäß der vorliegenden Erfindung weist noch die folgenden Elemente auf:
- eine medizintechnische Navigationsreferenz die positionsbestimmt gegenüber einer Bohrerführung angeordnet ist, die der Halterung zugeordnet ist,
- ein medizintechnisches Navigationssystem, das die Raumposition der Navigationsreferenz ermitteln kann,
- eine Unterdruckquelle, die mit der Unterdruck-Ansaugung des Befestigungsteils verbunden ist, und
- Steuervorrichtungen zum Steuern des Unterdrucks in Abhängigkeit von der Raumposition der Navigationsreferenz, und somit in Abhängigkeit von der Raumposition der Bohrerführung.

Mit der oben zuletzt genannten Ausführung lässt sich also eine erfindungsgemäße Knochenbohrerhalterung einfach und ohne weiteres in eine medizintechnische Navigation einordnen.

Die Erfindung wird im Weiteren anhand von Ausführungsbeispielen näher erläutert. Sie kann alle hierin beschriebenen Merkmale einzeln sowie in jedweder sinnvollen Kombination aufweisen. In den beiliegenden Zeichnungen zeigen:
- Figur 1: eine perspektivische Ansicht einer am Knochen angeordneten, erfindungsgemäßen Knochenbohrerhalterung;
- Figuren 2 bis 4: Knochenbohrerhalterungen gemäß der vorliegenden Erfindung in einer Schnittansicht und angeordnet am Knochen (Femur-Gelenkkopf);
- Figur 5 und 6: ein erfindungsgemäßes Knochenbohrer-Halterungssystem mit Handgriff und Unterdruckquelle; und
- Figur 7: die Einordnung eines erfindungsgemäßen Halterungssystems in ein medizintechnisches Navigationssystem.

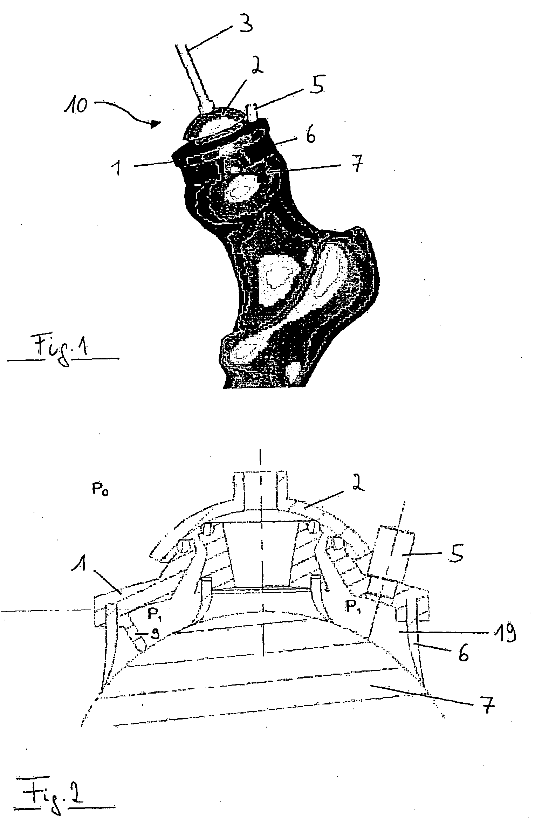

In den Figuren 1 bis 4 ist jeweils eine Knochenbohrerhalterung gemäß der vorliegenden Erfindung in einer Ausführungsform gezeigt, und insgesamt das Bezugszeichen 10 trägt. Sie weist einen Befestigungsteil 1 auf, der über dem nicht behandelten Gelenkkopf 7 eines Oberschenkelknochens sitzt. Berührt wird der Knochen nur mit den Dichtschürzen, die in den Figuren mit dem Bezugszeichen 6 angedeutet sind. In einem Kreisraum zwischen der inneren und äußeren Schürze 6 bildet sich ein Unterdruck, der den Befestigungsteil 1 auf dem Knochen 7 hält. Der Unterdruck ist als niedrigerer Druck in Figur 2 mit P₁ bezeichnet, während der höhere Außendruck dieser Zeichnung mit P₀ angegeben ist. Der höhere Außendruck P₀ drückt die Halterung 10 auf den Knochen 7, bis Stützstifte 9 zur Auflage kommen, von denen in den Figuren 2 bis 4 jeweils nur einer zu sehen ist.

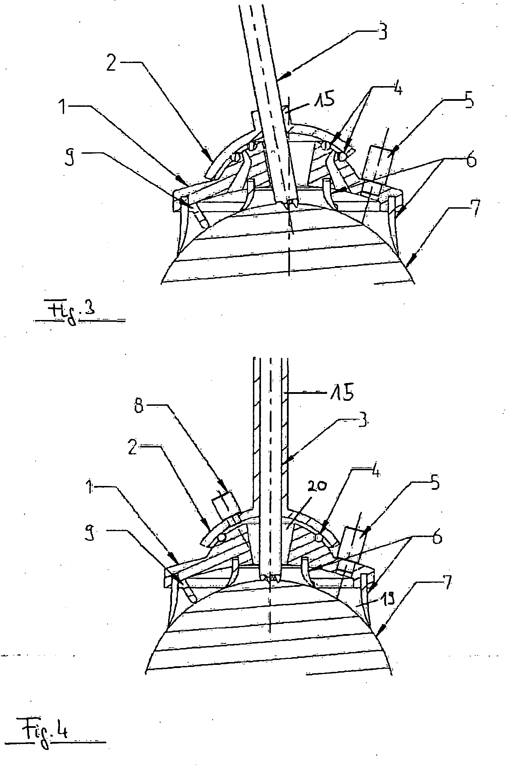

Auf Dichtringen 4 (Figuren 3 und 4) sitzt oben auf dem Befestigungsteil 1 der Führungsteil 2 der Halterung 10. Der Führungsteil 2 ist verschieblich angeordnet, und in den Figuren 2 und 3 sind zwei unterschiedliche Stellungen zu sehen. Durch die verschiebliche Anordnung kann der Bohrwinkel eingestellt werden; durch die Anordnung des Befestigungsteils 1 wird der Eindringpunkt ausgewählt und definiert.

Die Ausführungsform der Figuren 1, 2 und 3 hat ein einziges Ventil 5, mit dem aus dem Unterdruckraum 19 Luft abgesaugt werden kann, und bei dieser Ausführungsform wird der Befestigungsteil 1 positioniert, um den Eindringpunkt auszuwählen, der Führungsteil 2 wird verschoben bzw. gedreht, bis der Eindringwinkel stimmt, und dann wird über das Ventil 5 der Unterdruck aufgebracht, der sowohl den Befestigungsteil 1 als auch den Führungsteil 2 fixiert, so dass das Bohren dann definiert und richtig ausgerichtet stattfinden kann.

Eine etwas hiervon abweichende Ausführungsform ist in Figur 4 zu sehen, wobei zwei unabhängig voneinander arbeitende und separierte Unterdrucksysteme bereitgestellt werden. Das eine Unterdrucksystem beaufschlagt den Unterdruckraum 19 über das Ventil 5 zur Befestigung des Befestigungsteils 1, und das zweite Unterdrucksystem beaufschlagt über das Ventil 8 den Unterdruckraum 20 mit Unterdruck, um den Führungsteil 2 zu fixieren. Damit kann die Befestigung unabhängig von der Bohrerausrichtung stattfinden.

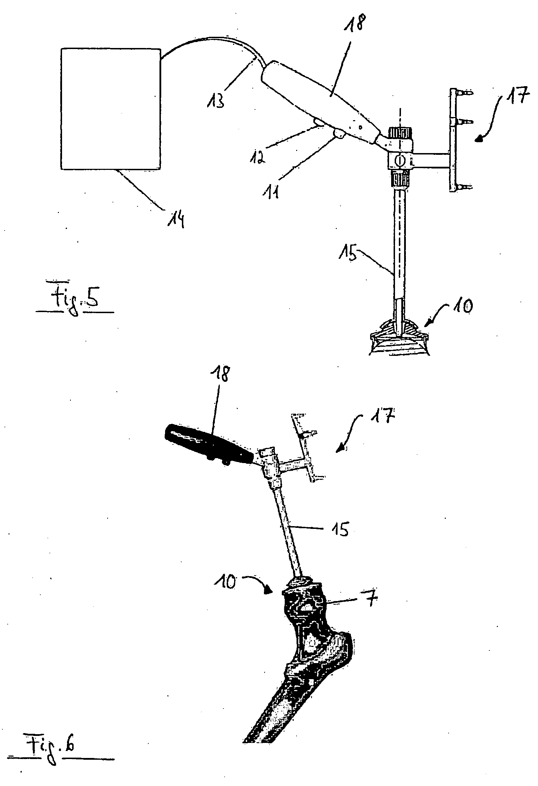

Eine Ausführungsform eines erfindungsgemäßen Knochenbohrer-Halterungssystems mit einer Unterdruckquelle 14 ist in den Figuren 5 und 6 dargestellt, wobei die Figur 6 die perspektivische Ansicht liefert. Bei der hier verwendeten Ausführungsform der Halterung 10 wird der Unterdruck direkt mittig über die Bohrerführung 15 in den entsprechenden Räumen der Halterung erzeugt. Die Bohrerführung 15 ist mit einer Navigationsreferenz 17 verbunden, auf die später noch eingegangen wird. Ferner ist an ihr der Handgriff 18 mit den beiden Druckknöpfen 11 und 12 angebracht, wobei der Handgriff eine Leitung 13 zu einer Unterdruckquelle 14 aufweist.

Über die Knöpfe 11 und 12 wird bei dieser Ausführungsform beispielsweise in der Knochenbohrer-Halterung 10 ein Vakuum erzeugt oder wieder aufgehoben. Mit dem Griff kann die Bohrerführung 15 ausgerichtet werden, bevor die Fixierung durch das Anlegen des Vakuums erfolgt.

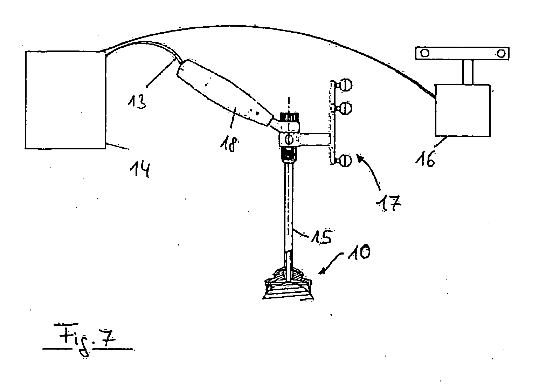

Mittels der vorher schon angesprochenen Navigationsreferenz 17 kann das Halterungssystem in ein medizintechnisches Navigationssystem eingebunden werden, und eine solche Einbindung ist in Figur 7 gezeigt. Dort ist zu sehen, dass das Navigationssystem 16 mit den nicht bezeichneten Trackingkameras so aufgestellt ist, dass es die Navigationsreferenz 17 in Position und Ausrichtung bestimmen kann. Wenn nun mittels des Griffs 18 die richtige Stellung für die Bohrhülse 15 gefunden worden ist, kann ein automatischer Ablauf durchgeführt werden, weil-wie dargestellt-das Navigationssystem 16 mit der Unterdruckquelle 14 verbunden ist. Wenn also die Ausrichtung der Bohrerführung 15 stimmt, wird dies durch das Navigationssystem 16 über die Navigationsreferenz 17 festgestellt, und diese Feststellung kann automatisch die Absaugung der Luft aus der Halterung 10 über die Bohrerführung 15, den Griff 18 und die Leitung 13 und mit Hilfe der Unterdruckquelle 14 auslösen. Dadurch wird sichergestellt, dass die Bohrung gerade in der Ausrichtung und Position erfolgt, die notwendig ist, um eine optimale spätere Implantatsetzung zu gestatten.

## Patentansprüche

1. Knochenbohrerhalterung (10) zum Anordnen eines Knochenbohrers (3) an einem Knochenteil (7), wobei die Halterung einen Befestigungsteil (1) zur Befestigung am Knochen aufweist, wobei der Befestigungsteil (1) mit einer Unterdruck-Ansaugung am Knochen befestigt wird, **dadurch gekennzeichnet, dass** am Befestigungsteil (1) ein Bohrerführungsteil (2) angeordnet ist, der gegenüber dem Befestigungsteil (1) beweglich ist.

2. Knochenbohrerhalterung (10) nach Anspruch 1, **dadurch gekennzeichnet, dass** der Befestigungsteil (1) einen Befestigungs-Unterdruckraum oder Befestigungs-Unterdruckbereich (19) aufweist, sowie eine Ventileinrichtung (5), über die der Druck im Befestigungs-Unterdruckraum oder Befestigungs-Unterdruckbereich reguliert werden kann.

3. Knochenbohrerhalterung (10) nach Anspruch 1 oder 2, **dadurch gekennzeichnet, dass** der Befestigungsteil (1) zur Abdichtung des Unterdrucks eine am Knochen angreifende Dichteinrichtung, insbesondere eine Dichtschürzenanordnung (6) um den Befestigungs-Unterdruckraum oder Befestigungs-Unterdruckbereich (19), aufweist.

4. Knochenbohrerhalterung (10) nach einem der Ansprüche 1 bis 3, **dadurch gekennzeichnet, dass** der Bohrerführungsteil (2) einen auf dem Befestigungsteil (1) verschieblichen und abgedichteten Aufsatz mit einer Bohrerführung (15) bildet.

5. Knochenbohrerhalterung (10) nach einem der Ansprüche 1 bis 4, **dadurch gekennzeichnet, dass** der Bohrerführungsteil (2) einen Führungs-Unterdruckraum oder Führungs-Unterdruckbereich (20) aufweist, der bei der Ausbildung eines Unterdrucks die Lage des Bohrerführungsteils gegenüber dem Befestigungsteil (1) fixiert.

6. Knochenbohrerhalterung (10) nach Anspruch 5, **dadurch gekennzeichnet, dass** die Befestigungs- und Führungs-Unterdruckräume oder -Unterdruckbereiche miteinander verbunden sind und einen einzigen Unterdruckbereich bzw. Unterdruckraum (19) bilden

7. Knochenbohrerhalterung (10) nach Anspruch 5, **dadurch gekennzeichnet, dass** die Unterdruckräume oder Unterdruckbereiche für die Befestigung (19) und die Führung (20) voneinander separiert sind, und insbesondere eineventileinrichtung (8) im Bohrerführungsteil (2) bereitgestellt wird, über die der Druck im Führungs-Unterdruckraum oder Führungs-Unterdruckbereich (20) reguliert werden kann.

8. Knochenbohrer-Halterungssystem mit einer Knochenbohrerhalterung (10) nach einem der Ansprüche 1 bis 7, und mit einem Handgriff (18), mit dem die Halterung (10) über eine Bohrerführung (15) gehalten werden kann, wobei insbesondere der Handgriff (18) mit einer Unterdruckquelle (14) und mit der Unterdruck-Ansaugung des Befestigungsteils (1) verbunden ist, und wobei dem Handgriff (18) Steuerungsvorrichtungen (11, 12) zum Steuern des Unterdrucks zugeordnet sind.

9. Knochenbohrer-Halterungssystem mit einer Knochenbohrerhalterung (10) nach einem der Ansprüche 1 bis 7, und mit den folgenden Elementen:
- einer medizintechnischen Navigationsreferenz (17) die positionsbestimmt gegenüber einer Bohrerführung (15) angeordnet ist, die der Halterung (10) zugeordnet ist,
- einem medizintechnischen Navigationssystem (16), das die Raumposition der Navigationsreferenz ermitteln kann,
- einer Unterdruckquelle (14), die mit der Unterdruck-Ansaugung des Befestigungsteils (1) verbunden ist, und
- mit Steuerungsvorrichtungen zum Steuern des Unterdrucks in Abhängigkeit von der Raumposition der Navigationsreferenz, und somit in Abhängigkeit von der Raumposition der Bohrerführung.

## Claims

1. A bone drill mounting (10) for arranging a bone drill (3) on a bone portion (7), wherein the mounting comprises a fastening portion (1) for fastening to the bone, wherein the fastening portion (1) is fastened to the bone using vacuum suction, **characterised in that** a drill guiding portion (2) is arranged on the fastening portion (1) and can be moved relative to the fastening portion (1).

2. The bone drill mounting (10) according to claim 1, **characterised in that** the fastening portion (1) comprises a fastening vacuum space or fastening vacuum area (19), and a valve means (5) via which the pressure in the fastening vacuum space or fastening vacuum area can be regulated.

3. The bone drill mounting (10) according to claim 1 or 2, **characterised in that** the fastening portion (1) comprises a sealing means which grips the bone, in particular a sealing skirt array (6) around the fastening vacuum space or fastening vacuum area (19), for sealing the vacuum.

4. The bone drill mounting (10) according to any one of claims 1 to 3, **characterised in that** the drill guiding portion (2) forms an attachment which is sealed and can be shifted on the fastening portion (1) and which comprises a drill guide (15).

5. The bone drill mounting (10) according to any one of claims 1 to 4, **characterised in that** the drill guiding portion (2) comprises a guiding vacuum space or guiding vacuum area (20) which fixes the position of the drill guiding portion relative to the fastening portion (1) when a vacuum is formed.

6. The bone drill mounting (10) according to claim 5, **characterised in that** the fastening and guiding vacuum spaces or vacuum areas are connected to each other and form a single vacuum area or vacuum space (19).

7. The bone drill mounting (10) according to claim 5, **characterised in that** the vacuum spaces or vacuum areas for fastening (19) and guiding (20) are separated from each other, and in particular a valve means (8) is provided in the drill guiding portion (2), via which the pressure in the guiding vacuum space or guiding vacuum area (20) can be regulated.

8. A bone drill mounting system comprising a bone drill mounting (10) according to any one of claims 1 to 7, and comprising a hand grip (18) using which the mounting (10) can be held via a drill guide (15), wherein in particular the hand grip (18) is connected to a vacuum source (14) and to the vacuum suction of the fastening portion (1), and wherein control devices (11, 12) for controlling the vacuum are assigned to the hand grip (18).

9. A bone drill mounting system comprising a bone drill mounting (10) according to any one of claims 1 to 7, and comprising the following elements:
- a medical navigation reference (17) which is arranged in a determined position relative to a drill guide (15) which is assigned to the mounting (10);
- a medical navigation system (16) which can ascertain the spatial position of the navigation reference;
- a vacuum source (14) which is connected to the vacuum suction of the fastening portion (1); and
- control devices for controlling the vacuum in accordance with the spatial position of the navigation reference, and thus in accordance with the spatial position of the drill guide.

## Revendications

1. Support (10) de perceuse pour os pour placer une perceuse pour os (3) sur une partie d'os (7), le support comportant une partie de fixation (1) pour la fixation sur l'os, la partie de fixation (1) étant fixée sur l'os par une aspiration à dépression, **caractérisé en ce que** sur la partie de fixation (1) est disposée une partie de guidage de perceuse (2) qui est mobile par rapport à la partie de fixation (1).

2. Support (10) de perceuse pour os selon la revendication 1, **caractérisé en que** la partie de fixation (1) comporte une chambre ou zone de fixation à dépression (19) ainsi qu'un dispositif à soupape (5) par lequel il est possible de réguler la pression dans la chambre ou zone de fixation.

3. Support (10) de perceuse pour os selon la revendication 1 ou 2, **caractérisé en que** la partie de fixation (1) comporte, pour l'étanchéité de la dépression, un dispositif d'étanchéité agissant sur l'os, en particulier un agencement à revêtement d'étanchéité (6) autour de la chambre ou zone de fixation à dépression (19).

4. Support (10) de perceuse pour os selon l'une quelconque des revendications 1 à 3, **caractérisé en que** la partie de guidage de perceuse (2) forme une garniture étanche et coulissante sur la partie de fixation (1), avec un guide de perceuse (15).

5. Support (10) de perceuse pour os selon l'une quelconque des revendications 1 à 4, **caractérisé en que** la partie de guidage de perceuse (2) comporte une chambre ou zone de guidage à dépression (20) qui fixe la position de la partie de guidage de perceuse par rapport à la partie de fixation (1) lors de la formation d'une dépression.

6. Support (10) de perceuse pour os selon la revendication 5, **caractérisé en que** les chambres ou zones de fixation et de guidage à dépression communiquent entre elles et forment respectivement une seule zone ou chambre à dépression (19).

7. Support (10) de perceuse pour os selon la revendication 5, **caractérisé en que** les chambres ou zones à dépression pour la fixation (19) et le guidage (20) sont séparées les unes des autres, et en particulier il est prévu un dispositif à soupape (8) dans la partie de guidage de perceuse (2) par lequel il est possible de réguler la pression dans la chambre ou zone de guidage à dépression (20).

8. Système de support de perceuse pour os avec un support (10) de perceuse pour os selon l'une quelconque des revendications 1 à 7, et avec une poignée (18) par laquelle il est possible de tenir le support (10) au-dessus d'un guide de perceuse (15), la poignée (18) communiquant en particulier avec une source de dépression (14) et avec l'aspiration à dépression de la partie de fixation (1), et des dispositifs de commande (11, 12) étant associés à la poignée (18) pour commander la dépression.

9. Système de support de perceuse pour os avec un support (10) de perceuse pour os selon l'une quelconque des revendications 1 à 7, et avec les éléments suivantes :
- une référence de navigation médicale (17) qui est disposée dans une position déterminée par rapport à un guide de perceuse (15) associé au support (10),
- un système de navigation médicale (16) qui peut fournir la position spatiale de la référence de navigation,
- une source de dépression (14) qui communique avec l'aspiration à dépression de la partie de fixation (1), et
- avec des dispositifs de commande pour commander la dépression en fonction de la position spatiale de la référence de navigation, et donc en fonction de la position spatiale du guide de perceuse.
